# FASCICULE DE BREVET EUROPEEN

(11) **EP 1 201 221 B1**
(45) Date de publication et mention de la délivrance du brevet: **10.01.2007**
(21) Numéro de dépôt: 01402490.5
(22) Date de dépôt: 27.09.2001
(51) Int. Cl.: A61K 8/04, A61K 8/64, A61K 8/73, A61K 8/81, A61K 8/85, A61K 8/87, A61K 8/88, A61K 8/92, A61Q 1/10

(54) **Composition cosmétique comprenant des fibres et une cire**
Fasern und ein Wachs enthaltende, kosmetische Zusammensetzung
Cosmetic composition containing fibres and a wax

(30) Priorité: 27.10.2000 FR 0013866
(43) Date de publication de la demande: 02.05.2002
(73) Titulaire: L'ORÉAL, 75008 Paris (FR)
(72) Inventeur: Piot, Bertrand, 75009 Paris (FR); Collin, Nathalie, 92330 Sceaux (FR)
(74) Mandataire: Kromer, Christophe

(56) Documents cités:
- EP-A- 1 031 342
- US-A- 5 743 949
- DATABASE CHEMICAL ABSTRACTS [en ligne] STN; numéro d'accès 97: 222 765, XP002171057 & JP 57 158714 A (SHISEIDO CO., LTD) 30 septembre 1982 (1982-09-30)

## Description

La présente invention a pour objet une composition comprenant, dans un milieu physiologiquement acceptable, des fibres et une cire, destinée en particulier au domaine cosmétique. L'invention a aussi pour objet un procédé de maquillage ou de soin cosmétique des matières kératiniques utilisant cette composition. La composition et le procédé selon l'invention sont plus particulièrement destinés aux matières kératiniques telles que la peau, y compris les lèvres, et les phanères comme les cils, les sourcils, les cheveux et les ongles, notamment d'êtres humains. Plus spécialement, l'invention porte sur un mascara.

La composition selon l'invention peut se présenter sous la forme de composition de revêtement des cils (notamment de mascara), d'eye-liner, de produit pour les lèvres, de fard à joues ou à paupières, de fond de teint, de produit de maquillage du corps, de produit anti-cernes, de vernis à ongles, de produit de soin de la peau.

Les produits de maquillage sont couramment employés pour apporter de la couleur, mettre en valeur certaines parties de la peau ou des phanères, ou bien encore procurer un aspect brillant, mat, satiné à la peau ou aux phanères. Ces produits sont habituellement appliqués sous la forme d'une mince couche uniforme.

Il est connu de l'état de la technique d'employer des fibres dans les compositions de maquillage ou de soin des matières kératiniques pour améliorer leurs propriétés cosmétiques.

Pour le maquillage des cils, le document JP-A-3-153613 enseigne l'utilisation des fibres dans des compositions de mascara pour conférer un effet d'allongement et d'épaississement aux cils. Les documents JP-A-6-9340 et JP-A-7-179323 décrivent des compositions de mascara comprenant des fibres et des polymères en dispersion aqueuse.

Pour le maquillage de la peau, il est connu du document JP-A-7-196440 d'utiliser des fibres pour conférer à la peau un toucher velouté.

Toutefois, lors de l'application de ces compositions sur les matières kératiniques, les fibres adhèrent difficilement sur les matières kératiniques. L'utilisatrice doit donc appliquer plusieurs fois la composition sur les matières kératiniques pour déposer une quantité suffisante de fibres pour obtenir les propriétés cosmétiques recherchées, ce qui nécessite de consacrer un certain temps pour se maquiller et obtenir les résultats de maquillage souhaités. Or ce temps peut être perçu comme trop long par les utilisatrices pressées. Un besoin existe donc de disposer d'une composition contenant des fibres permettant d'obtenir rapidement et facilement le maquillage attendu.

Par ailleurs, les fibres n'adhérant pas bien sur les matières kératiniques ont alors tendance à se détacher de leur support et s'éliminent donc dans le temps. L'élimination de ces fibres provoque alors une diminution perceptible des propriétés cosmétiques recherchées apportées par les fibres, nécessitant de renouveler l'application du produit. De plus, pour un mascara, les fibres en se détachant des cils peuvent se loger dans l'oeil et provoquer un inconfort occulaire.

Le but de la présente invention est de disposer d'une composition cosmétique comprenant des fibres adhérant bien sur les matières kératiniques.

La demanderesse a maintenant constaté de façon surprenante que l'utilisation d'une microdispersion de cire dans une composition comprenant des fibres permettait d'obtenir une composition qui s'applique facilement sur les matières kératiniques et permet de déposer rapidement les fibres sur les matières kératiniques. La composition appliquée sur les matières kératiniques sèche rapidement favorisant ainsi un maintient rapide et adhérant des fibres sur les matières kératiniques.

De plus, lorsque la composition est un mascara, on obtient un maquillage qui épaissit rapidement les cils et qui allonge bien les cils. On constate ainsi une charge instantanée des cils.

De façon plus précise, la présente invention a pour objet une composition comprenant, dans un milieu physiologiquement acceptable contenant au moins une phase aqueuse, des fibres, caractérisée par le fait qu'elle comprend une microdispersion aqueuse de particules de cire.

Un autre objet de l'invention est un mascara comprenant une composition telle que décrite précédemment.

L'invention a encore pour objet un produit de mascara comprenant un réservoir contenant une composition de mascara telle que définie précédemment, et muni d'un système d'application de la composition sur les fibres kératiniques, notamment les cils.

L'invention a aussi pour objet un procédé cosmétique de maquillage ou de soin des matières kératiniques comprenant l'application sur lesdites matières kératiniques d'une composition telle que définie précédemment. En particulier, l'invention a pour objet un procédé de revêtement des cils comprenant l'application sur les cils de la composition décrite précédemment.

L'invention a également pour objet l'utilisation d'une composition telle que définie précédemment pour obtenir un dépôt adhérant sur les matières kératiniques.

L'invention a encore pour objet l'utilisation, dans une composition comprenant, dans un milieu physiologiquement acceptable contenant au moins une phase aqueuse, de fibres et d'une microdispersion aqueuse de particules de cire, pour obtenir un dépôt adhérant sur les matières kératiniques.

Dans la présente demande, on entend par "milieu physiologiquement acceptable", un milieu compatible avec les matières kératiniques, comme un milieu cosmétique.

La phase aqueuse de la composition peut être constituée essentiellement d'eau. Elle peut comprendre également un mélange d'eau et de solvant miscible à l'eau comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en C₃-C₄, les aldéhydes en C₂-C₄. La phase aqueuse (eau et éventuellement le solvant organique miscible à l'eau) représente, en pratique, de 5 % à 99,4 % en poids, par rapport au poids total de la composition.

### a) les fibres :

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2 500, de préférence de 5 à 500, et mieux de 5 à 150.

Les fibres utilisables dans la composition de l'invention peuvent être des fibres d'origine synthétique ou naturelle, minérale ou organique. Elles peuvent être courtes ou longues, unitaires ou organisées par exemple tressées, creuses ou pleines. Leur forme peut être quelconque et notamment de section circulaire ou polygonale (carrée, hexagonale ou octogonale) selon l'application spécifique envisagée. En particulier, leurs extrémités sont épointées et/ou polies pour éviter de se blesser.

En particulier, les fibres ont une longueur allant de 1 µm à 10 mm, de préférence de 0,1 mm à 5 mm et mieux de 1 mm à 3,5 mm. Leur section peut être comprise dans un cercle de diamètre allant de 2 nm à 500 µm, de préférence allant de 100 nm à 100 µm et mieux de 1 µm à 50 µm. Le poids ou titre des fibres est souvent donné en denier ou décitex et représente le poids en gramme pour 9 km de fil. De préférence, les fibres selon l'invention ont un titre choisi dans la gamme allant de 0,15 à 30 deniers et mieux de 0,18 à 18 deniers.

Les fibres peuvent être celles utilisées dans la fabrication des textiles et notamment des fibres de soie, de coton, de laine, de lin, des fibres de cellulose - notamment extraites notamment du bois, des légumes ou des algues -, de rayonne, de polyamide (Nylon® ), de viscose, d'acétate notamment d'acétate de rayonne, de poly-(p-phénylène-téréphtalamide) (ou d'aramide) notamment de Kevlar® , de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène (comme le Téflon® ), de collagène insoluble, de polyesters, de polychlorure de vinyle ou de vinylidène, d'alcool polyvinylique, de polyacrylonitrile, de chitosane, de polyuréthane, de polyéthylène phtalate, des fibres formées d'un mélange de polymères tels que ceux mentionnés ci-avant, comme des fibres de polyamide/polyester.

On peut aussi utiliser les fibres utilisées en chirurgie comme les fibres synthétiques résorbables préparées à partir d'acide glycolique et caprolactone ("Monocryl" de chez Johnson & Johnson) ; les fibres synthétiques résorbables du type copolymère d'acide lactique et d'acide glycolique ("Vicryl" de chez Johnson & Johnson) ; les fibres de polyester téréphtalique ("Ethibond" de chez Johnson & Johnson) et les fils d'acier inoxydable ("Acier" de chez Johnson & Johnson) notamment pour une application en vernis à ongles.

Par ailleurs, les fibres peuvent être traités ou non en surface, enrobées ou non. Comme fibres enrobées utilisables dans l'invention, on peut citer des fibres de polyamides enrobées de sulfure de cuivre pour un effet anti-statique (par exemple le "R-STAT" de chez Rhodia) ou un autre polymère permettant une organisation particulière des fibres (traitement de surface spécifique) ou traitement de surface induisant des effets de couleurs/hologrammes (fibre "Lurex" de chez Sildorex, par exemple).

De préférence, on utilise des fibres d'origine synthétiques et en particulier des fibres organiques, comme celles utilisées en chirurgie. Avantageusement, on peut utiliser des fibres insolubles dans l'eau.

Les fibres utilisables dans la composition selon l'invention sont préférentiellement des fibres de polyamide, de cellulose, de poly-p-phénylène téréphtalamide ou de de polyéthylène. Leur longueur (L) peut aller de 0,1 mm à 5 mm, de préférence de 0,25 mm à 1,6 mm et leur diamètre moyen peut aller de 1 µm à 50 µm. En particulier, on peut utiliser les fibres de polyamide commercialisées par les Etablissements P. Bonte sous le nom de "Polyamide 0.9 Dtex 3 mm", ayant un diamètre moyen de 6 µm, un titre d'environ 0.9 dtex et une longueur allant de 0,3 mm à 5 mm. On peut aussi utiliser les fibres de poly-p-phénylène téréphtalamide de diamètre moyen de 12 µm et de longueur d'environ 1,5 mm comme celles vendues sous le nom de "Kevlar Floc" par la société Du Pont Fibres ou bien encore les fibres de celluloses (ou de rayonne) ayant un diamètre moyen de 50 µm et une longueur allant de 0,5 mm à 6 mm comme celles vendues sous le nom de "Natural rayon flock fiber RC1BE - N003 - M04" par la société Claremont Flock. On peut également utiliser des fibres de polyéthylène comme celles vendues sous le nom de "Shurt Stuff 13 099 F" par la société Mini Fibers.

Les fibres peuvent êtres présentes dans la composition selon l'invention en une teneur allant de 0,01 % à 10 % en poids, par rapport au poids total de la composition, de préférence de 0,1 % à 5 % en poids, et mieux de 0,3 % à 2 % en poids.

### b) la microdispersion de cire :

La composition selon l'invention comprend par ailleurs une microdispersion aqueuse de particules de cire. On entend par microdispersion aqueuse de cire, une dispersion aqueuse de particules de cire, dans laquelle la taille desdites particules de cire est inférieure ou égale à environ 1 µm.

Dans la présente demande, une cire est un composé lipophile, solide à température ambiante (25 °C), à changement d'état solide/liquide réversible, ayant un point de fusion supérieur ou égal à 30 °C pouvant aller jusqu'à 120 °C. En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

Le point de fusion de la cire peut être mesuré à l'aide d'un calorimètre à balayage différentiel (D.S.C.), par exemple le calorimètre vendu sous la dénomination DSC 30 par la société METLER. Un échantillon de 15 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température. Le point de fusion du composé est la valeur de la température correspondant au sommet du pic de la courbe représentant la variation de la différence de puissance absorbée en fonction de la température.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire. Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 µm (notamment allant de 0,02 µm à 1 µm), de préférence inférieures à 0,5 µm (notamment allant de 0,051 µm à 0,5 µm).
Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Les cires susceptibles d'être utilisées dans les compositions selon l'invention sont choisies parmi les cires, solides et rigides, à température ambiante d'origine animale, végétale, minérale ou de synthèse et leurs mélanges. De préférence, les cires entrant dans la composition peuvent présenter un point de fusion supérieur à 45°C environ, et en particulier supérieur à 55°C. La cire peut également présenter une dureté allant de 0,05 MPa à 15 MPa, et de préférende allant de 6 MPa à 15 MPa . La dureté est déterminée par la mesure de la force en compression mesurée à 20 °C à l'aide du texturomètre vendu sous la dénomination TA-TX2i par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm. Pour effectuer la mesure de dureté, la cire est fondue à une température égale au point de fusion de la cire + 20 °C. La cire fondue est coulée dans un récipient de 30 mm de diamètre et de 20 mm de profondeur. La cire est recristallisée à température ambiante (25 °C) pendant 24 heures, puis la cire est conservée pendant au moins 1 heure à 20 °C avant d'effectuer la mesure de dureté. La valeur de la dureté est la force de compression mesurée divisée par la surface du cylindre du texturomètre en contact avec la cire.

On peut notamment citer les cires hydrocarbonées comme la cire d'abeilles, la cire de lanoline, et les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch et les copolymères cireux ainsi que leurs esters.
On peut aussi citer les cires obtenues par hydrogénation catalytique d'huiles animales ou végétales ayant des chaînes grasses, linéaires ou ramifiées, en C8-C32. Parmi celles-ci, on peut notamment citer l'huile de jojoba hydrogénée, l'huile de tournesol hydrogénée, l'huile de ricin hydrogénée, l'huile de coprah hydrogénée et l'huile de lanoline hydrogénée.

On peut encore citer les cires de silicone, les cires fluorées.

Il est également possible d'utiliser des mélanges commerciaux de cires auto-émulsionnables contenant une cire et des tensioactifs. On peut utiliser par exemple la cire commercialisée sous la dénomination 'Cire Auto Lustrante OFR' par Tiscco, qui contient des cires de Carnauba et de paraffine en association avec des tensioactifs non ioniques, ou la cire auto-émulsionnable commercialisée sous la dénomination 'Cerax A.O. 28/B' par La Ceresine, qui contient de la cire d'Alfa en association avec un tensioactif non ionique. Ces mélanges commerciaux permettent de préparer des microdispersions de cires par simple addition d'eau.
On peut encore citer les produits 'Aquacer' de Byk Cera, et notamment : le mélange de cires synthétiques et naturelles avec émulsionnant anionique (Aquacer 520), la cire de polyéthylène avec émulsionnant non ionique ( Aquacer 514 ou 513), la cire polymérique avec émulsionnant anionique (Aquacer 511). On peut également citer le mélange de cires de polyéthylène et de paraffine avec émulsionnant non ionique ' Jonwax 120' de Johnson Polymer.

La cire peut être présente dans la composition selon l'invention en une teneur en matière sèche allant de 0,1 % à 50 % en poids, par rapport au poids total de la composition, de préférence de 0,5 % à 30 % en poids, et mieux de 1 % à 20 % en poids.

La composition peut également comprendre une quantité suffisante de tensioactif pour permettre d'obtenir une microdispersion de cire, ainsi qu'une composition finale, stable. Notamment, elle peut comprendre 0,01 % à 30% en poids de tensioactif usuel, pouvant être choisi parmi les composés suivants :
- les tensioactifs anioniques, notamment des sels d'acides gras éventuellement insaturés, ayant par exemple 12 à 18 atomes de carbone; des sels alcalins ou sels de bases organiques des acides alkyl-sulfuriques et alkylsulfoniques ayant 12 à 18 atomes de carbone ou d'acides alkyl-arylsulfoniques dont la chaîne alkyle contient 6-18 atomes de carbone; les éthers-sulfates.
- les tensioactifs non ioniques, notamment des tensioactifs polyalcoxylés et/ou polyglycérolés, et en particulier des acides gras ou amides d'acide gras; des alcools gras ou des alkylphénols; les esters d'acides gras et de polyols; les alcanediols et les alkyléthers d'alcanediols. On peut citer également les alkylcarbamates de triglycérol, les dérivés oxyéthylénés ou propoxylés des alcools de lanoline, des acides gras de la lanoline, ou de leur mélanges.
- les tensioactifs cationiques, notamment les dérivés d'ammonium quaternaire.

La cire ou mélange de cires, peut être associé à un ou plusieurs additifs gras (huileux et/ou pâteux). On peut notamment citer les huiles végétales comme l'huile de tournesol, l'huile de jojoba; les huiles minérales comme l'huile de paraffine; les huiles de silicones; la vaseline, la lanoline; les huiles fluorées; les huiles hydrocarbonées à groupement perfluoré; les esters d'alcools gras.

Il est possible d'introduire en outre dans la phase cireuse microparticulaire des ingrédients actifs liposolubles, tels que des filtres U.V., des vitamines liposolubles, des actifs cosmétiques liposolubles.

### c) le polymère filmogène :

La composition selon l'invention peut comprendre au moins un polymère filmogène, notamment pour obtenir un film présentant une bonne tenue, en particulier un film résistant à l'eau ( comme par exemple la pluie, les larmes, l'eau de baignade) et/ou aux frottements et/ou au sébum.

Le polymère filmogène peut être un polymère solubilisé ou dispersé sous forme de particules dans la phase aqueuse de la composition ou bien encore solubilisé ou dispersé sous forme de particules dans une phase grasse liquide. La composition peut comprendre un mélange de ces polymères.

Le polymère filmogène peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Dans la présente demande, on entend par "polymère filmogène", un polymère apte à former à lui seul ou en présence d'un agent auxiliaire de filmification, un film continu et adhérent sur un support, notamment sur les matières kératiniques.

On utilise de préférence un polymère filmogène apte à former un film hydrophobe, c'est-à-dire un polymère dont le film a une solubilité dans l'eau à 25 °C inférieure à 1 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).

Les polymères filmogènes de type radicalaire peuvent être notamment des polymères, ou des copolymères, vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés α,β-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliques et les monomères styrèniques. En particulier, ces monomères peuvent être polymérisés avec des monomères acides et/ou leurs esters et/ou leurs amides, tels que ceux mentionnés précédemment.

Comme exemple d'esters vinyliques, on peut citer l'acétate de vinyle, le néodécanoate de vinyle, le pivalate de vinyle, le benzoate de vinyle et le t-butyl benzoate de vinyle.
Comme monomères styrèniques, on peut citer le styrène et l'alpha-méthyl styrène.

La liste des monomères donnée n'est pas limitative et il est possible d'utiliser tout monomère connu de l'homme du métier entrant dans les catégories de monomères acryliques et vinyliques (y compris les monomères modifiés par une chaîne siliconée).

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les polyuréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.

L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexa-nedicarboxylique, l'acide isophtalique, l'acide téréphtalique, l'acide 2,5-norborane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol.
Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement -SO₃M, avec M représentant un atome d'hydrogène, un ion ammonium NH₄⁺ ou un ion métallique, comme par exemple un ion Na⁺, Li⁺, K⁺, Mg²⁺, Ca²⁺, Cu²⁺, Fe²⁺, Fe³⁺. On peut utiliser notamment un monomère aromatique bifonctionnel comportant un tel groupement -SO₃M.

Le noyau aromatique du monomère aromatique bifonctionnel portant en outre un groupement -SO₃M tel que décrit ci-dessus peut être choisi par exemple parmi les noyaux benzène, naphtalène, anthracène, diphényl, oxydiphényl, sulfonyldiphényl, méthylènediphényl. On peut citer comme exemple de monomère aromatique bifonctionnel portant en outre un groupement -SO₃M : l'acide sulfoisophtalique, l'acide sulfotéréphtalique, l'acide sulfophtalique, l'acide 4-sulfonaphtalène-2,7-dicarboxylique.
On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique. De tels polymères sont vendus par exemple sous le nom de marque Eastman AQ® par la société Eastman Chemical Products.

Les polymères d'origine naturelle, éventuellement modifiés, peuvent être choisis parmi la résine shellac, la gomme de sandaraque, les dammars, les élémis, les copals, les polymères cellulosiques insolubles dans l'eau, et leurs mélanges.

Selon un premier mode de réalisation de la composition selon l'invention, le polymère filmogène peut être présent sous la forme de particules en dispersion aqueuse, connue généralement sous le nom de latex ou pseudolatex. Les techniques de préparation de ces dispersions sont bien connues de l'homme du métier.
La composition présente l'avantage de former un dépôt sur les matières kératiniques résistant à l'eau froide et pouvant se démaquiller facilement à l'eau chaude, c'est à dire une eau portée à une température supérieure ou égale à 35 °C, et notamment allant de 35 °C à 50 °C. Le dépôt s'enlève facilement avec de l'eau chaude ne contenant pas d'agent détergent tel que les savons.

Comme dispersion aqueuse de polymère filmogène, on peut utiliser les dispersions acryliques vendues sous les dénominations NEOCRYL XK-90® , NEOCRYL A-1070® , NEOCRYL A-1090® , NEOCRYL BT-62® , NEOCRYL A-1079® , NEOCRYL A-523® par la société AVECIA-NEORESINS, DOW LATEX 432® par la société DOW CHEMICAL, DAITOSOL 5000 AD® par la société DAITO KASEY KOGYO; ou ben encore les dispersions aqueuses de polyuréthane vendues sous les dénominations NEOREZ R-981® , NEOREZ R-974® par la société AVECIA-NEORESINS, les AVALURE UR-405® , AVALURE UR-410® , AVALURE UR-425 ® , AVALURE UR-450® , SANCURE 875® , SANCURE 861® , SANCURE 878® , SANCURE 2060® par la société GOODRICH, IMPRANIL 85® par la société BAYER, AQUAMERE H-1511® par la société HYDROMER.

Comme dispersion aqueuse de polymère filmogène, on peut également utiliser les dispersions de polymères résultant de la polymérisation radicalaire d'un ou plusieurs monomères radicalaires à l'intérieur et/ou partiellement en surface, de particules préexistantes d'au moins un polymère choisi dans le groupe constitué par les polyuréthanes, les polyurées, les polyesters, les polyesteramides et/ou les alkydes. Ces polymères sont généralement appelés polymères hybrides.

Selon une deuxième variante de réalisation de la composition selon l'invention, le polymère filmogène peut être un polymère hydrosoluble et est donc présent dans la phase aqueuse de la composition sous forme solubilisée. Comme exemples de polymères filmogènes hydrosolubles, on peut citer
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères ou copolymères acryliques, tels que les polyacrylates ou les polyméthacrylates ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :
   . les gommes arabiques, la gomme de guar, les dérivés du xanthane, la gomme de karaya ;
   . les alginates et les carraghénanes ;
   . les glycoaminoglycanes, l'acide hyaluronique et ses dérivés ;
   . la résine shellac, la gomme de sandaraque, les dammars, les élémis, les copals ;
   . l'acide désoxyribonucléïque ;
   . les muccopolysaccharides tels que l'acide hyaluronique, les chondroïtines sulfate, et leurs mélanges.

Selon une autre variante de réalisation de la composition selon l'invention, le polymère filmogène peut être présent dans une phase grasse liquide.
De préférence, la phase grasse liquide comprend une phase grasse liquide volatile, éventuellement en mélange avec une phase grasse liquide non volatile.

Par "phase grasse volatile", on entend tout milieu non aqueux susceptible de s'évaporer de la peau en moins d'une heure. Cette phase volatile comporte notamment des huiles ayant une pression de vapeur, à température ambiante et pression atmosphérique allant de 10⁻³ à 300 mm de Hg (0,13 Pa à 40 000 Pa).

La phase grasse liquide peut être constituée de toute huile physiologiquement acceptable et en particulier cosmétiquement acceptable, notamment choisie parmi les huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée.

La phase grasse liquide totale de la composition peut représenter de 5 % à 98 % en poids, par rapport au poids total de la composition et de préférence de 20 à 85 % en poids. La partie non volatile peut représenter de 0 à 80 % (notamment de 0,1 à 80 %) du poids total de la composition et mieux de 1 à 50%.

Comme phase grasse liquide utilisable dans l'invention, on peut ainsi citer les esters d'acide gras, les acides gras supérieurs, les alcools gras supérieurs, les polydiméthylsiloxane (PDMS), éventuellement phénylées telles que les phényltriméthicones, ou éventuellement substitués par des groupements aliphatiques et/ou aromatiques, éventuellement fluorés, ou par des groupements fonctionnels tels que des groupements hydroxyle, thiol et/ou amine ; les polysiloxanes modifiés par des acides gras, des alcools gras ou des polyoxyalkylènes, les silicones fluorées, les huiles perfluorées.

Avantageusement, on peut utiliser une ou plusieurs huiles volatiles à température ambiante. Après évaporation de ces huiles, on obtient un dépôt filmogène souple, non collant. Ces huiles volatiles facilitent, en outre, l'application de la composition sur les matières kératiniques.

Ces huiles volatiles peuvent être des huiles hydrocarbonées, des huiles siliconées comportant éventuellement des groupements alkyle ou alkoxy en bout de chaîne siliconée ou pendante.

Comme huile de silicone volatile utilisable dans l'invention, on peut citer les silicones linéaires ou cycliques ayant de 2 à 7 atomes de silicium, ces silicones comportant éventuellement des groupes alkyle ou alkoxy ayant de 1 à 10 atomes de carbone. on peut citer notamment l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

Comme huile hydrocarbonée volatile, on peut citer les isoparaffines en C₈-C₁₆ telles que les 'ISOPARs', les PERMETYLs et notamment l'isododécane.

Ces huiles volatiles peuvent être présentes dans la composition en une teneur allant de 5 à 94,9 % du poids total de la composition, et mieux de 20 à 85 %.

Des huiles pouvant être utilisées dans la phase grasse liquides sont par exemples citées dans la demande EP-A-749747. Comme milieu non aqueux, on peut aussi utiliser ceux décrits dans le document FR-A-2 710 646 de L.V.M.H.
Selon un troisième mode de réalisation de la composition selon l'invention, le polymère filmogène peut être présent sous forme de particules, stabilisées en surface, dispersées dans la phase grasse liquide.

La dispersion de particules de polymère stabilisées en surface peut être fabriquée comme décrit dans le document EP-A-749747. Elle peut être obtenue par polymérisation en dispersion, c'est-à-dire par précipitation du polymère en cours de formation, avec protection des particules formées avec un stabilisant.

Le choix de la phase grasse liquide est effectué par l'homme du métier en fonction de la nature des monomères constituant le polymère et/ou de la nature du stabilisant, comme indiqué ci-après.

Les particules de polymère sont stabilisées en surface grâce à un stabilisant qui peut être un polymère séquencé, un polymère greffé, et/ou un polymère statistique, seul ou en mélange.

Parmi les polymères greffés, on peut citer les polymères siliconés greffés avec une chaîne hydrocarbonée ; les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également utiliser des copolymères blocs greffés ou séquencés comprenant au moins un bloc de type polyorganosiloxane et au moins un bloc d'un polymère radicalaire, comme les copolymères greffés de type acrylique/silicone qui peuvent être employés notamment lorsque le milieu non aqueux est siliconé.

Le stabilisant peut aussi être choisi parmi les copolymères blocs greffés ou séquencés comprenant au moins un bloc de type polyorganosiloxane et au moins d'un polyéther. Le bloc polyorganopolysiloxane peut être notamment un polydiméthylsiloxane ou bien encore un poly alkyl(C₂-C₁₈) méthyl siloxane ; le bloc polyéther peut être un poly alkylène en C₂-C₁₈, en particulier polyoxyéthylène et/ou polyoxypropylène. En particulier, on peut utiliser les diméthicones copolyol ou encore les alkyl (C₂-C₁₈) méthicones copolyol. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".

Comme copolymères blocs greffés ou séquencés, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly-(méthylacrylate de méthyle)/polyisobutylène ou les copolymères greffés à squelette poly(méthylacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également employer des copolymères de (méth)acrylates d'alkyl en C₁-C₄, et de (méth)acrylates d'alkyl en C₈-C₃₀. On peut en particulier citer le copolymère méthacrylate de stéaryle/méthacrylate de méthyle.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque la phase grasse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxy (C₂-C₁₈)alkylène et notammment polyoxypropyléné et/ou oxyéthyléné.

Lorsque la phase grasse liquide ne comprend pas d'huile de silicone, l'agent stabilisant est de préférence choisi dans le groupe constitué par :
- (a) les copolymères blocs greffés ou séquencés comprenant au moins un bloc de type polyorganosiloxane et au moins un bloc d'un polymère radicalaire ou d'un polyéther ou d'un polyester,
- (b) les copolymères d'acrylates ou de méthacrylates d'alkyl en C₁-C₄, et d'acrylates ou de méthacrylates d'alkyl en C₈-C₃₀,
- (c) les copolymères blocs greffés ou séquencés comprenant au moins un bloc résultant de la polymérisation d'au moins un monomère éthylénique, à liaisons éthyléniques conjuguées,
   et au moins un bloc d'un polymère vinylique ou acrylique ou d'un polyéther ou d'un polyester, ou leurs mélanges.

De préférence, on utilise des polymères dibloc comme agent stabilisant.

Des dispersions de polymère filmogène dans la phase grasse liquide, en présence d'agent stabilisants, sont notamment décrites dans les documents EP-A-749746, EP-A-923928, EP-A-930060 dont le contenu est incorporé à titre de référence dans la présente demande.

La taille des particules de polymères en dispersion soit dans la phase aqueuse, soit dans la phase grasse liquide, peut aller de 5 nm à 600 nm, et de préférence de 20 nm à 300 nm.

Selon un quatrième mode de réalisation de la composition selon l'invention, le polymère filmogène peut être solubilisé dans la phase grasse liquide, on dit alors que le polymère filmogène est un polymère liposoluble.
A titre d'exemple de polymère liposoluble, on peut citer les polymères correspondant à la formule (I) suivante : 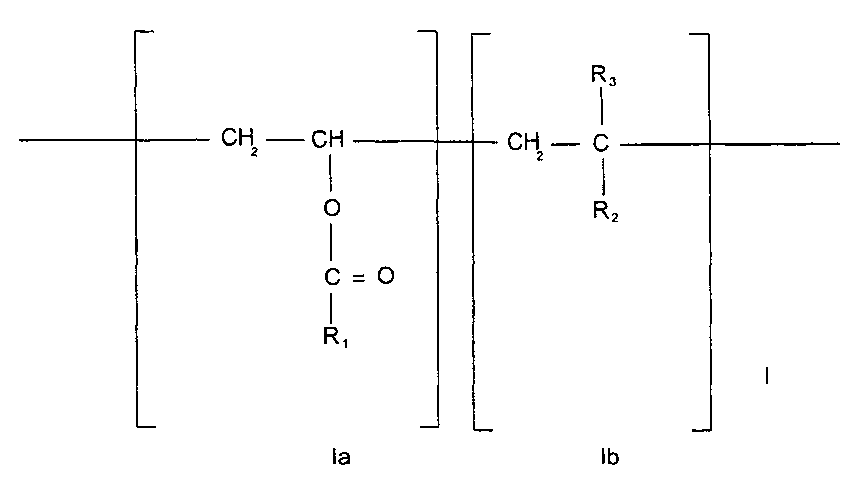 dans laquelle :
- R₁ représente une chaîne hydrocarbonée saturée, linéaire ou ramifiée, ayant de 1 à 19 atomes de carbone ;
- R₂ représente un radical pris dans le groupe constitué par :
   a) -O-CO-R₄, R₄ ayant la même signification que R₁ mais est différent de R₁ dans un même copolymère,
   b) -CH₂-R₅, R₅ représentant une chaîne hydrocarbonée saturée, linéaire ou ramifiée, ayant de 5 à 25 atomes de carbone,
   c) -O-R₆, R₆ représentant une chaîne hydrocarbonée saturée, ayant de 2 à 18 atomes de carbone, et
   d) -CH₂-O-CO-R₇, R₇ représentant une chaîne hydrocarbonée saturée, linéaire ou ramifiée, ayant de 1 à 19 atomes de carbone,
- R₃ représente un atome d'hydrogène quand R₂ représente les radicaux a), b) ou c) ou R₃ représente un radical méthyle quand R₂ représente le radical d), ledit copolymère devant être constitué d'au moins 15 % en poids d'au moins un monomère dérivé d'un motif (la) ou d'un motif (Ib) dans lesquels les chaînes hydrocarbonées, saturées ou ramifiées, ont au moins 7 atomes de carbone.

Les copolymères de formule (I) résultent de la copolymérisation d'au moins un ester vinylique (correspondant au motif la) et d'au moins un autre monomère (correspondant au motif Ib) qui peut être une α-oléfine, un alkylvinyléther ou un ester allylique ou méthallylique.

Lorsque dans le motif (Ib) R₂ est choisi parmi les radicaux -CH₂-R₅, -O-R₆ ou -CH₂-O-CO-R₇ tels que définis précédemment, le copolymère de formule (I) peut être constitué de 50 à 95 % en moles d'au moins un motif (la) et de 5 à 50 % en moles d'au moins un motif (Ib).

Les copolymères de formule (I) peuvent également résulter de la copolymérisation d'au moins un ester vinylique et d'au moins un autre ester vinylique différent du premier. Dans ce cas, ces copolymères peuvent être constitués de 10 à 90 % en moles d'au moins un motif (la) et de 10 à 90% en moles d'au moins un motif (Ib) dans lequel R₂ représente le radical -O-CO-R₄.

Parmi les esters vinyliques conduisant au motif de formule (la), ou au motif de formule (Ib) dans lequel R₂ = -O-CO-R₄, on peut citer l'acétate de vinyle, le propionate de vinyle, le butanoate de vinyle, l'octanoate de vinyle, le décanoate de vinyle, le laurate de vinyle, le stéarate de vinyle, l'isostéarate de vinyle, le diméthyl-2, 2 octanoate de vinyle, et le diméthylpropionate de vinyle.

Parmi les α-oléfines conduisant au motif de formule (Ib) dans lequel R₂ = -CH₂-R₅, on peut citer l'octène-1, le dodécène-1, l'octadécène-1, l'eicosène-1, et les mélanges d' α-oléfines ayant de 22 à 28 atomes de carbone.

Parmi les alkylvinyléthers conduisant au motif de formule (Ib) dans lequel R₂ = -O-R₆, on peut citer l'éthylvinyléther, le n-butylvinyléther, l'isobutylvinyléther, le décylvinyléther, le dodécylvinyléther, le cétylvinyléther et l'octadécylvinyléther.

Parmi les esters allyliques ou méthallyliques conduisant au motif de formule (Ib) dans lequel R₂ = -CH₂-O-CO-R₇, on peut citer les acétates, les propionates, les diméthylpropionates, les butyrates, les hexanoates, les octanoates, les décanoates, les laurates, les diméthyl-2, 2 pentanoates, les stéarates et les eicosanoates d'allyle et de méthallyle.

Les copolymères de formule (I) peuvent également être réticulés à l'aide de certains types de réticulants qui ont pour but d'augmenter sensiblement leur poids moléculaire.

Cette réticulation est effectuée lors de la copolymérisation et les réticulants peuvent être soit du type vinylique, soit du type allylique ou méthallylique. Parmi ceux-ci, on peut citer en particulier le tétraallyloxyéthane, le divinylbenzène, l'octanedioate de divinyle, le dodécanedioate de divinyle, et l'octadécanedioate de divinyle.

Parmi les différents copolymères de formule (I) utilisables dans la composition selon l'invention, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les homopolymères liposolubles, et en particulier ceux résultant de l'homopolymérisation d'esters vinyliques ayant de 9 à 22 atomes de carbone ou d'acrylates ou de méthacrylates d'alkyle, les radicaux alkyles ayant de 10 à 20 atomes de carbone.

De tels homopolymères liposolubles peuvent être choisis parmi le polystéarate de vinyle, le polystéarate de vinyle réticulé à l'aide de divinylbenzène, de diallyléther ou de phtalate de diallyle, le poly(méth)acrylate de stéaryle, le polylaurate de vinyle, le poly(méth)acrylate de lauryle, ces poly(méth)acrylates pouvant être réticulés à l'aide de diméthacrylate de l'éthylène glycol ou de tétraéthylène glycol.

Les copolymères et homopolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en C₂-C₂₀, différents de la cire de polyoléfine définie en a), comme le polybutène, les alkylcelluloses avec un radical alkyle linéaire ou ramifié, saturé ou non en C₁ à C₈ comme l'éthylcellulose et la propylcellulose, les copolymères de la vinylpyrolidone (VP) et notamment les copolymères de la vinylpyrrolidone et d'alcène en C₂ à C₄₀ et mieux en C₃ à C₂₀. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

La composition selon l'invention peut comprendre un agent auxiliaire de filmification favorisant la formation d'un film avec le polymère filmogène. Un tel agent de filmification peut être choisi parmi tous les composés connus de l'homme du métier comme étant susceptibles de remplir la fonction recherchée, et notamment être choisi parmi les agents plastifiants et les agents de coalescence.

### d) les additifs :

La composition peut également comprendre au moins une matière colorante comme les composés pulvérulents et/ou les colorants liposolubles, par exemple à raison de 0,01 à 30 % du poids total de la composition. Les composés pulvérulents peuvent être choisis parmi les pigments et/ou les nacres, autres que celles décrites précédemment, habituellement utilisés dans les compositions cosmétiques ou dermatologiques. Avantageusement, les composés pulvérulents représentent de 0,1 à 25 % du poids total de la composition et mieux de 1 à 20 %.

Les pigments peuvent être blancs ou colorés, minéraux et/ou organiques. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

La composition selon l'invention peut également contenir des ingrédients couramment utilisés en cosmétique, tels que les charges, les vitamines, les oligoéléments, les adoucissants, les séquestrants, les parfums, les diméthicone copolyols, les céramides, les agents de cohésion ainsi que les agents alcalinisants ou acidifiants habituellement utilisés dans le domaine cosmétique, les conservateurs, les épaississants, les agents plastifiants, les agents de coalescence, les matières colorantes, les cires, les tensio actifs, les conservateurs, les huiles, les agents hydratants ou leurs mélanges.

Bien entendu, l'homme du métier veillera à choisir ce ou ces éventuels composés complémentaires, et/ou leur quantité, de manière telles que les propriétés avantageuses de la composition selon l'invention ne soient pas, ou substantiellement pas, altérées par l'adjonction envisagée.

La composition selon l'invention peut être préparée de façon connue pour l'homme du métier par simple mélange des ingrédients, et en incorporant à la fin la microdispersion de cire à température ambiante ou à une température allant jusqu'à 45 °C, puis en terminant par ajouter les fibres.

La composition selon l'invention peut être une composition de soin ou de traitement de la peau, notamment le camouflage des imperfections de la peau, notamment pour le visage, le cou, les mains, ou le corps, ou bien encore une composition de maquillage telle que mascara, fond-de-teint, fard-à-joues, fard-à-paupières, produit pour sourcils, eye-liner, produit à appliquer sur les lèvres, composition de maquillage du corps (du type tatouage provisoire ou semi-permanent), vernis à ongles.

En particulier, la composition selon l'invention peut être destinée à un produit de mascara comprenant un réservoir, contenant ladite composition de mascara, et un système d'application de ladite composition sur les fibres kératiniques, notamment les cils. Le réservoir est muni de façon connue d'une ouverture dans laquelle est logée un système d'essorage. Le système d'application comporte une tige munie à une première extrémité d'une brosse et à une deuxième extrémité d'un bouchon destiné à fermer le réservoir. Un tel conditionnement est notamment illustré à la figure 7 de la demande EP-A-611170 qui est incorporée à titre de référence.

L'invention est illustrée plus en détail dans les exemples suivants.

### Exemple 1 :

On a préparé une microdispersion de cire de carnauba ayant la composition suivante :
- Cire de carnauba 27 g
- Monostéarate de glycéryle polyoxyéthyléné (30 OE) (TAGAT S de GOLDSCHMIDT) 6,75 g
- Ethanol 10 g
- Eau qsp 100 g

On a chauffé à 90 °C la cire et le tensioactif en homogénéisant le mélange sous agitation modérée. Puis on a incorporé l'eau chauffée à 90 °C en continuant d'agiter. On a refroidit à température ambiante et ajouté l'éthanol pour obtenir une microdispersion de cire ayant un diamètre moyen de particules d'environ 170 nm.

### Exemple 2 :

On a préparé un mascara ayant la composition suivante :
- Microdispersion de cire de l'exemple 1 36 g
- Sulfopolyester (AQ 55S de la société EASTMAN KODAK) 22 g
- Fibres de polyamide (3 mm de long et 0,9 Dtex de la société Paul Bonte) 0,5 g
- Hydroxyéthylcellulose 0,6 g
- Propylène glycol 3 g
- Oxyde de fer noir 5 g
- Conservateurs qs
- Eau qsp 100 g

La composition appliquée sur les cils sèche rapidement et adhère très bien sur ceux-ci et les allonge visiblement. Le maquillage présente une bonne tenue en atmosphère humide ( 30 °C à 80 % d'humidité relative) et se démaquille facilement par simple application d'un coton imbibé d'eau tiède. Le mascara s'élimine proprement sous forme de gaine sans laiser de trace, ni d'auréoles sur les paupières.

### Exemple 3 :

On a préparé un mascara ayant la composition suivante :
- Cire de carnauba 5,5 g
- Cire de son de riz 2,6 g
- Paraffine 2,6 g
- Cire d'abeille 9,7 g
- Microdispersion de cire de l'exemple 1 6,7 g
- Fibres de polyamide (3 mm de long et 0,9 Dtex de la société Paul Bonte) 1 g
- Talc 1 g
- Bentonite 5,5 g
- Copolymère acétate de vinyle/stéarate d'allyle (65/35) 7,4 g
- Polylaurate de vinyle (Mexomère PP de CHIMEX) 0,7 g
- Isododécane 50,3 g
- Carbonate de propylène 1,7 g
- D-panthénol 0,2 g
- Pigments 4,9 g
- Conservateurs qs
- Eau qsp 100 g

Le mascara s'applique facilement sur les cils et conduit à un maquillage allongeant bien adhérant sur les cils.

## Revendications

1. Composition comprenant, dans un milieu physiologiquement acceptable contenant au moins une phase aqueuse, des fibres, **caractérisée par le fait qu**'elle comprend une microdispersion aqueuse de particules de cire.

2. Composition selon la revendication précédente, **caractérisée par le fait que** les fibres sont choisies parmi les fibres de soie, de coton, de laine, de lin, des fibres de cellulose, de polyamide, de rayonne, de viscose, d'acétate notamment d'acétate de rayonne, de poly-(p-phénylène-téréphtalamide), de polymère acrylique notamment de polyméthacrylate de méthyle ou de poly 2-hydroxyéthyl méthacrylate, de polyoléfine et notamment de polyéthylène ou de polypropylène, de verre, de silice, de carbone notamment sous forme graphite, de polytétrafluoroéthylène, de collagène insoluble, de polyesters, de polychlorure de vinyle ou de vinylidène, d'alcool polyvinylique, de polyacrylonitrile, de chitosane, de polyuréthane, de polyéthylène phtalate, des fibres de mélanges de polymères.

3. Composition selon l'une quelconque des revendications précédentes, **caractérisée par le fait que** les fibres sont des fibres d'origine synthétique.

4. Composition selon l'une quelconque des revendications précédentes, **caractérisée par le fait que** les fibres sont des fibres de polyamide, de poly-(p-phénylène-téréphtalamide), de cellulose ou de polyéthylène.

5. Composition selon l'une quelconque des revendications précédentes, **caractérisée en ce que** les fibres ont une longueur L et un diamètre D tel que L/D est choisi dans la gamme allant de 3,5 à 2 500, de préférence de 5 à 500, et mieux de 5 à 150.

6. Composition selon l'une quelconque des revendications précédentes, **caractérisée par le fait que** les fibres ont une longueur allant de 1 µm à 10 mm, de préférence de 0,1 mm à 5 mm, et mieux de 1 mm à 3,5 mm.

7. Composition selon l'une quelconque des revendications précédentes, **caractérisée par le fait que** les fibres ont un titre choisi dans la gamme allant de 0,15 à 30 deniers et mieux de 0,18 à 18 deniers.

8. Composition selon l'une quelconque des revendications précédentes, **caractérisée par le fait que** les fibres ont une section comprise dans un cercle de diamètre moyen allant de 2 nm à 500 µm, de préférence allant de 100 nm à 100 µm, et mieux allant de 1 µm à 50 µm.

9. Composition selon l'une quelconque des revendications précédentes, **caractérisée par le fait que** les fibres sont présentes en une teneur allant de 0,01 % à 10 % en poids, par rapport au poids total de la composition, de préférence de 0,1 % à 5 % en poids, et mieux de 0,3 % à 2 % en poids.

10. Composition selon l'une quelconque des revendications précédentes, **caractérisée par le fait que** la microdispersion de cires comprend des particules de cires ayant une taille moyenne inférieure à 1 µm, de préférence inférieure à 0,5 µm.

11. Composition selon l'une quelconque des revendications précédentes, **caractérisée par le fait que** la cire est choisie dans le groupe formé par les cires ayant un point de fusion allant de 30 °C à 120 °C.

12. Composition selon l'une quelconque des revendications précédentes, **caractérisée par le fait que** la cire a une dureté allant de 0,05 MPa à 15 MPa.

13. Composition selon l'une des revendications précédentes, **caractérisée par le fait que** la cire est choisie dans le groupe formé par la cire d'abeilles, la cire de lanoline, les cires d'insectes de Chine; la cire de riz, la cire de Carnauba, la cire de Candellila, la cire d'Ouricurry, la cire d'Alfa, la cire de fibres de liège, la cire de canne à sucre, la cire du Japon et la cire de sumac; la cire de montan, les cires microcristallines, les paraffines et l'ozokérite; les cires de polyéthylène, les cires obtenues par la synthèse de Fisher-Tropsch; les copolymères cireux ainsi que leurs esters; les cires obtenues par hydrogénation catalytique d'huiles animales ou végétales ayant des chaînes grasses, linéaires ou ramifiées, en C8-C32; l'huile de jojoba hydrogénée, l'huile de tournesol hydrogénée, l'huile de ricin hydrogénée, l'huile de coprah hydrogénée, l'huile de lanoline hydrogénée; les cires de silicone; leurs mélanges.

14. Composition selon l'une des revendications précédentes, **caractérisée par le fait que** la cire est présente en une teneur en matière sèche allant de 0,1 % à 50 % en poids, par rapport au poids total de la composition, de préférence allant de 0,5 % à 30% en poids, et mieux allant de 1 % à 20 % en poids.

15. Composition selon l'une des revendications précédentes, dans laquelle les particules de la dispersion de cire comprennent en outre des additifs gras huileux et/ou pâteux et/ou un additif/actif liposoluble.

16. Composition selon l'une des revendications précédentes, comprenant en outre au moins un tensioactif.

17. Composition selon l'une quelconque des revendications précédentes, **caractérisée par le fait qu**'elle comprend un polymère filmogène.

18. Composition selon la revendication 17, **caractérisée par le fait que** le polymère filmogène est choisi dans le groupe formé par les polymères radicalaires, les polycondensats, les polymères d'origine naturelle et leurs mélanges.

19. Composition selon l'une des revendications 17 ou 18, **caractérisée par le fait que** le polymère filmogène est choisi dans le groupe formé par les polymères vinyliques résultant de la polymérisation de monomères choisis parmi les acides carboxyliques insaturés α,β-éthyléniques, les esters de cesdits acides, les amides de cesdits acides, les esters vinyliques, les monomères styréniques.

20. Composition selon l'une des revendications 17 à 19, **caractérisée par le fait que** le polymère filmogène est choisi dans le groupe formé par les polyuréthanes, les polyesters, les polyesters amides, les polyamides, les résines époxyesters, les polyurées.

21. Composition selon l'une des revendications 17 à 20, **caractérisée par le fait que** le polymère filmogène est choisi dans le groupe formé par la résine shellac, la gomme de sandaraque, les dammars, les élémis, les copals, les polymères cellulosiques insolubles dans l'eau, et leurs mélanges.

22. Composition selon l'une quelconque des revendications 17 à 21, **caractérisée par le fait que** le polymère filmogène est présent en une teneur en matière sèche allant de 0,1 % à 60 % en poids, par rapport au poids total de la composition, de préférence allant de 0,5 % à 40% en poids, et mieux allant de 1 % à 30 % en poids.

23. Composition selon l'une quelconque des revendications 17 à 22, **caractérisée par le fait que** le polymère filmogène est sous forme de particules dispersées dans la phase aqueuse.

24. Composition selon l'une quelconque des revendications 17 à 22, **caractérisée par le fait que** le polymère filmogène est solubilisé ou dispersé sous forme de particules stabilisées en surface dans une phase grasse liquide.

25. Composition selon la revendication 24, **caractérisée par le fait que** la phase grasse liquide comprend une phase grasse liquide volatile.

26. Composition selon la revendication 24 ou 25, **caractérisée par le fait que** les particules de polymère filmogène sont stabilisées en surface par un stabilisant.

27. Composition selon l'une quelconque des revendications précédentes, **caractérisée par le fait que** la composition comprend, en outre, au moins un additif choisi dans le groupe formé par les épaississants, les agents plastifiants, les agents de coalescence, les charges, les matières colorantes, les cires, les tensio-actifs, les conservateurs, les huiles, les agents hydratants, les parfums, et leurs mélanges.

28. Mascara comprenant une composition selon l'une quelconque des revendications précédentes.

29. Produit de mascara comprenant un réservoir, contenant une composition de mascara , et un système d'application de ladite composition sur les fibres kératiniques, **caractérisé par le fait que** la composition est une composition selon l'une quelconque des revendications 1 à 27.

30. Procédé cosmétique de traitement ou de maquillage des matières kératiniques comprenant l'application sur lesdites matières kératiniques d'une composition selon l'une quelconque des revendications 1 à 27.

31. Procédé de revêtement des cils comprenant l'application sur les cils d'une composition telle que définie selon l'une quelconque des revendications précédentes.

32. Utilisation d'une composition telle que définie selon l'une quelconque des revendications 1 à 27 pour obtenir un dépôt adhérant sur les matières kératiniques.

33. Utilisation, dans une composition comprenant, dans un milieu physiologiquement acceptable contenant au moins une phase aqueuse, de fibres et d'une microdispersion aqueuse de particules de cire, pour obtenir un dépôt adhérant sur les matières kératiniques.

34. Utilisation selon la revendication 33, **caractérisée par le fait que** la composition comprend un polymère filmogène.

35. Utilisation d'une composition selon la revendication 34 pour obtenir un dépôt sur les matières kératiniques résistant à l'eau froide et se démaquillant à l'eau chaude.

## Claims

1. Composition comprising fibres, in a physiologically acceptable medium containing at least one aqueous phase, **characterized in that** it comprises an aqueous microdispersion of particles of wax.

2. Composition according to the preceding claim, **characterized in that** the fibres are chosen from silk fibre, cotton fibre, wool fibre, flax fibre, cellulose fibres, polyamide fibre, rayon fibre, viscose fibre, acetate fibre, in particular rayon acetate fibre, poly(p-phenylene-terephthalamide) fibre, acrylic polymer fibre, in particular polymethyl methacrylate fibre or poly-2-hydroxyethyl methacrylate fibre, polyolefin fibre and in particular polyethylene or polypropylene fibre, glass fibre, silica fibre, carbon fibre, in particular in graphite form, polytetrafluoroethylene fibre, insoluble collagen fibre, polyester fibre, polyvinyl or polyvinylidene chloride fibre, polyvinyl alcohol fibre, polyacrylonitrile fibre, chitosan fibre, polyurethane fibre, polyethylene phthalate fibre, fibres consisting of mixtures of polymers.

3. Composition according to any one of the preceding claims, **characterized in that** the fibres are fibres of synthetic origin.

4. Composition according to any one of the preceding claims, **characterized in that** the fibres are polyamide, poly(p-phenylene-terephthalamide), cellulose or polyethylene fibres.

5. Composition according to any one of the preceding claims, **characterized in that** the fibres have a length L and a diameter D such that L/D is chosen from the range of from 3.5 to 2500, preferably from 5 to 500, and better still from 5 to 150.

6. Composition according to any one of the preceding claims, **characterized in that** the fibres have a length ranging from 1 µm to 10 mm, preferably from 0.1 mm to 5 mm, and better still from 1 mm to 3.5 mm.

7. Composition according to any one of the preceding claims, **characterized in that** the fibres have a titre chosen from the range of from 0.15 to 30 denier and better still from 0.18 to 18 denier.

8. Composition according to any one of the preceding claims, **characterized in that** the fibres have a section contained in a circle having a mean diameter ranging from 2 nm to 500 µm, preferably ranging from 100 nm to 100 µm, and better still ranging from 1 µm to 50 µm.

9. Composition according to any one of the preceding claims, **characterized in that** the fibres are present in an amount ranging from 0.01% to 10% by weight, relative to the total weight of the composition, preferably from 0.1% to 5% by weight, and better still from 0.3% to 2% by weight.

10. Composition according to any one of the preceding claims, **characterized in that** the microdispersion of waxes comprises particles of waxes having a mean size of less than 1 µm, preferably less than 0.5 µm.

11. Composition according to any one of the preceding claims, **characterized in that** the wax is chosen from the group formed by waxes having a melting point ranging from 30°C to 120°C.

12. Composition according to any one of the preceding claims, **characterized in that** the wax has a hardness ranging from 0.05 MPa to 15 MPa.

13. Composition according to one of the preceding claims, **characterized in that** the wax is chosen from the group formed by beeswax, lanolin wax, Chinese waxes; rice wax, Carnauba wax, candelilla wax, ouricury wax, Esparto wax, cork fibre wax, sugarcane wax, Japan wax and sumac wax; montan wax, microcrystalline waxes, paraffins and ozokerite; polyethylene waxes, waxes obtained by Fisher-Tropsch synthesis; waxy copolymers as well as their esters; the waxes obtained by catalytic hydrogenation of animal or vegetable oils having linear or branched C₈-C₃₂ fatty chains; hydrogenated jojoba oil, hydrogenated sunflower oil, hydrogenated castor oil, hydrogenated copra oil and hydrogenated lanolin oil; silicone waxes; mixtures thereof.

14. Composition according to one of the preceding claims, **characterized in that** the wax is present in a dry matter content ranging from 0.1% to 50% by weight, relative to the total weight of the composition, preferably ranging from 0.5% to 30% by weight, and better still from 1% to 20% by weight.

15. Composition according to one of the preceding claims, in which the particles of the wax dispersion comprise, in addition, oily and/or pasty fatty additives and/or a fat-soluble additive/active agent.

16. Composition according to one of the preceding claims, comprising, in addition, at least one surfactant.

17. Composition according to any one of the preceding claims, **characterized in that** it comprises a film-forming polymer.

18. Composition according to Claim 17, **characterized in that** the film-forming polymer is chosen from the group formed by free-radical polymers, polycondensates, polymers of natural origin and mixtures thereof.

19. Composition according to either of Claims 17 and 18, **characterized in that** the film-forming polymer is chosen from the group formed by the vinyl polymers resulting from the polymerization of monomers chosen from α,β-ethylenic unsaturated carboxylic acids, esters of the said acids, amides of the said acids, vinyl esters and styrene monomers.

20. Composition according to one of Claims 17 to 19, **characterized in that** the film-forming polymer is chosen from the group formed by polyurethanes, polyesters, polyester amides, polyamides, epoxy ester resins and polyureas.

21. Composition according to one of Claims 17 to 20, **characterized in that** the film-forming polymer is chosen from the group formed by shellac resin, sandarac gum, dammars, elemis, copals, water-insoluble cellulosic polymers and mixtures thereof.

22. Composition according to any one of Claims 17 to 21, **characterized in that** the film-forming polymer is present in a dry matter content ranging from 0.1% to 60% by weight, relative to the total weight of the composition, preferably ranging from 0.5% to 40% by weight, and better still ranging from 1% to 30% by weight.

23. Composition according to any one of Claims 17 to 22, **characterized in that** the film-forming polymer is in the form of particles dispersed in the aqueous phase.

24. Composition according to any one of Claims 17 to 22, **characterized in that** the film-forming polymer is solubilized or dispersed in the form of surface-stabilized particles in a liquid fatty phase.

25. Composition according to Claim 24, **characterized in that** the liquid fatty phase comprises a volatile liquid fatty phase.

26. Composition according to Claim 24 or 25, **characterized in that** the particles of film-forming polymer are surface-stabilized by a stabilizer.

27. Composition according to any one of the preceding claims, **characterized in that** the composition comprises, in addition, at least one additive chosen from the group formed by thickeners, plasticizing agents, coalescing agents, fillers, colouring matter, waxes, surfactants, preservatives, oils, moisturizing agents, perfumes and mixtures thereof.

28. Mascara comprising a composition according to any one of the preceding claims.

29. Mascara product comprising a reservoir, containing a mascara composition, and a system for applying the said composition to the keratinous fibres, **characterized in that** the composition is a composition according to any one of Claims 1 to 27.

30. Cosmetic method for treating or applying make-up to the keratinous materials comprising the application to the said keratinous materials of a composition according to any one of Claims 1 to 27.

31. Method for covering the eyelashes comprising the application to the eyelashes of a composition as defined in any one of the preceding claims.

32. Use of a composition as defined according to any one of Claims 1 to 27 for obtaining a deposit adhering to the keratinous materials.

33. Use, in a composition comprising, in a physiologically acceptable medium containing at least one aqueous phase, fibres and an aqueous microdispersion of particles of wax, for obtaining a deposit adhering to the keratinous materials.

34. Use according to Claim 33, **characterized in that** the composition comprises a film-forming polymer.

35. Use of a composition according to Claim 34 for obtaining a deposit on the keratinous materials which is resistant to cold water and which can be removed as make-up using hot water.

## Patentansprüche

1. Zusammensetzung, die in einem physiologisch akzeptablen Medium, das mindestens eine wässrige Phase aufweist, Fasern enthält, **dadurch gekennzeichnet, dass** sie eine wässrige Mikrodispersion von Wachspartikeln enthält.

2. Zusammensetzung nach dem vorhergehenden Anspruch, **dadurch gekennzeichnet, dass** die Fasern unter den Seidenfasern, Baumwollfasern, Wollfasern, Leinenfasem, Cellulosefasern, Polyamidfasern, Rayonfasern, Viskosefasern, Acetatfasern und insbesondere Fasern aus Rayonacetat, Poly-(*p*-phenylenterephthalamid)-Fasern, Acrylfasern und insbesondere Fasern aus Polymethylmethacrylat oder Poly-2-hydroxyethylmethacrylat, Polyolefinfasern und insbesondere Fasern aus Polyethylen oder Polypropylen, Glasfasern, Siliciumoxidfasern, Kohlenstofffasern, insbesondere Fasern aus in Form von Graphit vorliegendem Kohlenstoff, Polytetrafluorethylenfasern, Fasern aus unlöslichem Collagen, Polyesterfasern, Polyvinylchloridfasern, Polyvinylidenchloridfasern, Polyvinylalkoholfasern, Polyacrylnitrilfasern, Chitosanfasern, Polyurethanfasern, Polyethylenphthalatfasern und Fasern aus Polymergemischen ausgewählt sind.

3. Zusammensetzung nach einem der vorhergehenden Ansprüche, **dadurch gekennzeichnet, dass** die Fasern Fasern synthetischer Herkunft sind.

4. Zusammensetzung nach einem der vorhergehenden Ansprüche, **dadurch gekennzeichnet, dass** es sich bei den Fasern um Polyamidfasern, Poly-(*p*-phenylenterephthalamid)-Fasern, Cellulosefasern oder Polyethylenfasern handelt.

5. Zusammensetzung nach einem der vorhergehenden Ansprüche, **dadurch gekennzeichnet, dass** die Fasern eine Länge L und einen Durchmesser D aufweisen, die so sind, dass L/D im Bereich von 3,5 bis 2 500, vorzugsweise 5 bis 500 und besser 5 bis 150 liegt.

6. Zusammensetzung nach einem der vorhergehenden Ansprüche, **dadurch gekennzeichnet, dass** die Fasern eine Länge von 1 µm bis 10 mm, vorzugsweise 0,1 bis 5 mm und besser 1 bis 3,5 mm aufweisen.

7. Zusammensetzung nach einem der vorhergehenden Ansprüche, **dadurch gekennzeichnet, dass** die Fasern einen Titer aufweisen, der im Bereich von 0,15 bis 30 Denier und besser 0,18 bis 18 Denier liegt.

8. Zusammensetzung nach einem der vorhergehenden Ansprüche, **dadurch gekennzeichnet, dass** der Querschnitt der Fasern in einen Kreis mit einem mittleren Durchmesser von 2 nm bis 500 µm, vorzugsweise 100 nm bis 100 µm und besser 1 bis 50 µm einbeschrieben werden kann.

9. Zusammensetzung nach einem der vorhergehenden Ansprüche, **dadurch gekennzeichnet, dass** die Fasern in einer Menge von 0,01 bis 10 Gew.-%, bezogen auf das Gesamtgewicht der Zusammensetzung, vorzugsweise 0,1 bis 5 Gew.-% und besser 0,3 bis 2 Gew.-% enthalten sind.

10. Zusammensetzung nach einem der vorhergehenden Ansprüche, **dadurch gekennzeichnet, dass** die Wachsmikrodispersion Wachspartikel enthält, die eine mittlere Größe unter 1 µm und vorzugsweise unter 0,5 µm aufweisen.

11. Zusammensetzung nach einem der vorhergehenden Ansprüche, **dadurch gekennzeichnet, dass** das Wachs unter den Wachsen ausgewählt ist, die einen Schmelzpunkt von 30 bis 120 °C aufweisen.

12. Zusammensetzung nach einem der vorhergehenden Ansprüche, **dadurch gekennzeichnet, dass** das Wachs eine Härte von 0,05 bis 15 MPa hat.

13. Zusammensetzung nach einem der vorhergehenden Ansprüche, **dadurch gekennzeichnet, dass** das Wachs unter Bienenwachs, Lanolinwachs, Chinawachs; Reiswachs, Carnaubawachs, Candelillawachs, Ouricurywachs, Alfawachs, Korkfaserwachs, Zuckerrohrwachs, Japanwachs und Sumachwachs; Montanwachs, mikrokristallinen Wachsen, Paraffinen und Ozokerit; Polyethylenwachsen, Wachsen, die durch Fischer-Tropsch-Synthese hergestellt werden; wachsigen Copolymeren sowie deren Estern; Wachsen, die durch katalytische Hydrierung von tierischen oder pflanzlichen Ölen hergestellt werden, die geradkettige oder verzweigte Fettketten mit 8 bis 32 Kohlenstoffatomen aufweisen; hydriertem Jojobaöl, hydriertem Sonnenblumenöl, hydriertem Ricinusöl, hydriertem Kopraöl, hydriertem Lanolinöl; Siliconwachsen; und deren Gemischen ausgewählt ist.

14. Zusammensetzung nach einem der vorhergehenden Ansprüche, **dadurch gekennzeichnet, dass** das Wachs in einem Trockensubstanzgehalt von 0,1 bis 50 Gew.-%, bezogen auf das Gesamtgewicht der Zusammensetzung, vorzugsweise 0,5 bis 30 Gew.-% und besser 1 bis 20 Gew.-% enthalten ist.

15. Zusammensetzung nach einem der vorhergehenden Ansprüche, bei der die Partikel der Wachsdispersion ferner ölige und/ oder pastöse Fettsubstanzen und/oder einen fettlöslichen Zusatzstoff/Wirkstoff enthalten.

16. Zusammensetzung nach einem der vorhergehenden Ansprüche, die ferner mindestens einen grenzflächenaktiven Stoff enthält.

17. Zusammensetzung nach einem der vorhergehenden Ansprüche, **dadurch gekennzeichnet, dass** sie ein filmbildendes Polymer aufweist.

18. Zusammensetzung nach Anspruch 17, **dadurch gekennzeichnet, dass** das filmbildende Polymer unter den durch radikalische Polymerisation erhaltenen Polymeren, Polykondensaten, Polymeren natürlicher Herkunft und deren Gemischen ausgewählt ist.

19. Zusammensetzung nach einem der Ansprüche 17 oder 18, **dadurch gekennzeichnet, dass** das filmbildende Polymer unter den Vinylpolymeren, die bei der Polymerisation von Monomeren gebildet werden, die unter den α-β-ethylenisch ungesättigten Carbonsäuren, Estern dieser Säuren, Amiden dieser Säuren, Vinylestern und Styrolmonomeren ausgewählt ist.

20. Zusammensetzung nach einem der Ansprüche 17 bis 19, **dadurch gekennzeichnet, dass** das filmbildende Polymer unter den Polyurethanen, Polyestern, Polyesteramiden, Polyamiden, Epoxyesterharzen und Polyharnstoffen ausgewählt ist.

21. Zusammensetzung nach einem der Ansprüche 17 bis 20, **dadurch gekennzeichnet, dass** das filmbildende Polymer unter Schellack, Sandarak, Dammarharzen, Elemi, Kopalen, in Wasser unlöslichen Cellulosepolymeren und deren Gemischen ausgewählt ist.

22. Zusammensetzung nach einem der Ansprüche 17 bis 21, **dadurch gekennzeichnet, dass** das filmbildende Polymer in einem Trockensubstanzgehalt von 0,1 bis 60 Gew.-%, bezogen auf das Gesamtgewicht der Zusammensetzung, vorzugsweise 0,5 bis 40 Gew.-% und besser 1 bis 30 Gew.-% enthalten sein kann.

23. Zusammensetzung nach einem der Ansprüche 17 bis 22, **dadurch gekennzeichnet, dass** das filmbildende Polymer in Form von in der wässrigen Phase dispergierten Partikeln vorliegt.

24. Zusammensetzung nach einem der Ansprüche 17 bis 22, **dadurch gekennzeichnet, dass** das filmbildende Polymer in Form von an der Oberfläche stabilisierten Partikeln in einer flüssigen Fettphase solubilisiert oder dispergiert ist.

25. Zusammensetzung nach Anspruch 24, **dadurch gekennzeichnet, dass** die flüssige Fettphase eine flüchtige flüssige Fettphase umfasst.

26. Zusammensetzung nach Anspruch 24 oder 25, **dadurch gekennzeichnet, dass** die Partikel des filmbildenden Polymers an der Oberfläche durch einen Stabilisator stabilisiert sind.

27. Zusammensetzung nach einem der vorhergehenden Ansprüche, **dadurch gekennzeichnet, dass** die Zusammensetzung ferner mindestens einen Zusatzstoff enthält, der unter den Verdickungsmitteln, Weichmachern, Koaleszenzmitteln, Füllstoffen, Farbmitteln, Wachsen, grenzflächenaktiven Stoffen, Konservierungsmitteln, Ölen, Hydratisierungsmitteln, Parfums und deren Gemischen ausgewählt ist.

28. Mascara, die eine Zusammensetzung nach einem der vorhergehenden Ansprüche enthält.

29. Mascaraprodukt, das ein Reservoir mit einer Mascarazusammensetzung und ein System zum Aufragen der Zusammensetzung auf die Keratinfasern umfasst, **dadurch gekennzeichnet, dass** die Zusammensetzung eine Zusammensetzung nach einem der Ansprüche 1 bis 27 ist.

30. Kosmetisches Verfahren zur Behandlung oder zum Schminken von Keratinsubstanzen, das das Auftragen einer Zusammensetzung nach einem der Ansprüche 1 bis 27 auf die Keratinsubstanzen umfasst.

31. Verfahren zum Überziehen von Wimpern, das das Aufbringen einer Zusammensetzung nach einem der vorhergehenden Ansprüche auf die Wimpern umfasst.

32. Verwendung einer Zusammensetzung nach einem der Ansprüche 1 bis 27, um eine auf den Keratinsubstanzen haftende Abscheidung zu bilden.

33. Verwendung von Fasern und einer wässrigen Mikrodispersion von Wachspartikeln in einer Zusammensetzung, die ein physiologisch akzeptables Medium enthält, das mindestens eine wässrige Phase aufweist, um eine auf den Keratinsubstanzen haftende Abscheidung zu bilden.

34. Verwendung nach Anspruch 33, **dadurch gekennzeichnet, dass** die Zusammensetzung ein filmbildendes Polymer enthält.

35. Verwendung einer Zusammensetzung nach Anspruch 34, um auf den Keratinsubstanzen eine Abscheidung zu bilden, die gegenüber kaltem Wasser beständig ist und sich mit heißem Wasser abnehmen lässt.
